(19) Europäisches Patentamt
European Patent Office
Office européen des brevets

(11) EP 0 880 966 A1

(12) **EUROPEAN PATENT APPLICATION**
published in accordance with Art. 158(3) EPC

(43) Date of publication:
**02.12.1998 Bulletin 1998/49**

(21) Application number: **96938466.8**

(22) Date of filing: **14.11.1996**

(51) Int. Cl.$^6$: **A61K 31/505**, C07D 239/42

(86) International application number:
**PCT/JP96/03341**

(87) International publication number:
**WO 98/20877 (22.05.1998 Gazette 1998/20)**

(84) Designated Contracting States:
**AT BE CH DE DK ES FI FR GB GR IE IT LI LU MC NL PT SE**

(71) Applicant:
**NISSUI PHARMACEUTICAL CO., LTD.**
**Toshima-ku, Tokyo 170 (JP)**

(72) Inventors:
- **SHOJI, Shozo**
  **Kumamoto-shi, Kumamoto 862 (JP)**
- **TACHIBANA, Kuniomi**
  **Yuuki-shi, Ibaraki 307 (JP)**

(74) Representative:
**Hartz, Nikolai F., Dr. et al**
**Wächtershäuser & Hartz,**
**Patentanwälte,**
**Tal 29**
**80331 München (DE)**

(54) **THIAMINE DISULFIDES AND MEDICINES CONTAINING THE SAME AS THE ACTIVE INGREDIENT**

(57) The invention relates to an anti-HIV agent and a prophylactic and therapeutic agent for AIDS, each comprising thiamine disulfide dimyristate or a salt thereof as an active ingredient. The compound has an excellent anti-HIV effect and is useful as a prophylactic and therapeutic agent for AIDS.

EP 0 880 966 A1

## Description

### TECHNICAL FIELD

The present invention relates to thiamine disulfide derivatives which have an anti-HIV effect and are useful for prophylaxis and treatment of acquired immune deficiency syndrome (AIDS).

### BACKGROUND ART

AIDS is a disease caused by infection with HIV (human immunodeficiency virus), and the number of patients thereof is rapidly increasing since this disease was discovered in the United States of America in 1983. It has been known to use azidothymidine (AZT), didanosine (DDI) or the like, which is an anti-HIV agent, in treatment for such AIDS.

However, AZT is recognized to have a life-prolonging effect to a significant extent, but involves a problem that headache, gastrointestinal disorders, myelodepresant effect and the like are caused as its side effects. Besides, since many of these anti-HIV agents, which have heretofore been studied, have been based on the action mechanism that DNA synthesis in the replication process of HIV is inhibited to suppress the proliferation of HIV, they have involved a problem that the DNA synthesis of normal cells is also suppressed at the same time as the inhibition of HIV, and normal cells of patients treated with the anti-HIV agents hence decrease, and consequently, the patients still more fall into a dangerous condition.

It is accordingly an object of the present invention to provide a novel compound which is excellent in safety and anti-HIV effect and useful as a therapeutic agent for AIDS.

### DISCLOSURE OF THE INVENTION

In view of the foregoing circumstances, the present inventors have screened anti-HIV effects of various compounds in accordance with a screening method of anti-HIV agents by Tat (transactivator) inhibition, which has recently been developed. As a result, it has been found that a novel compound obtained by myristoylating thiamine disulfide, which is widely used as a vitamin $B_1$ derivative, exhibits an anti-HIV effect by an action mechanism which is not found in the existing anti-HIV agents and is useful for prophylaxis and treatment of AIDS, thus leading to completion of the present invention.

According to the present invention, there is thus provided thiamine disulfide dimyristate represented by the following formula (1):

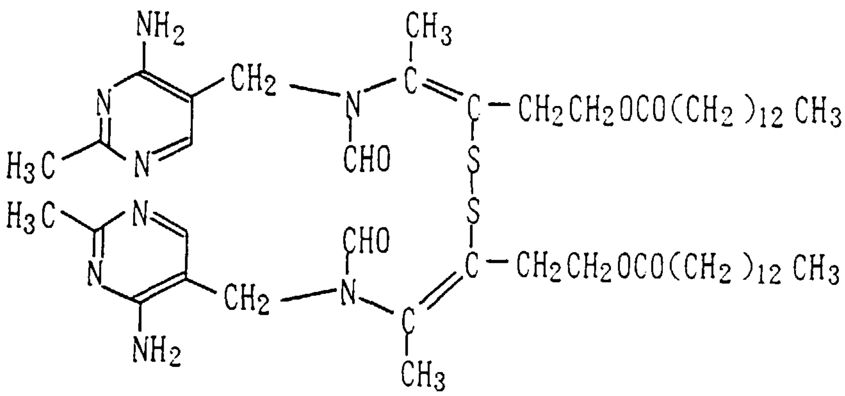

(1)

or a salt thereof.

According to the present invention, there are also provided an anti-HIV agent, a prophylactic and therapeutic agent for AIDS and compositions thereof, which each comprise thiamine disulfide dimyristate (1) or the salt thereof as an active ingredient.

According to the present invention, there is further provided use of thiamine disulfide dimyristate (1) or the salt thereof for the preparation of an anti-HIV agent and of a prophylactic and therapeutic agent for AIDS.

According to the present invention, there is still further provided a method for the treatment of a disease caused by infection with HIV and of AIDS, which comprises administering an effective amount of thiamine disulfide dimyristate (1) or the salt thereof to a patient to be treated.

## BRIEF DESCRIPTION OF THE DRAWING

FIG. 1 is a drawing illustrating a nuclear magnetic resonance spectrum of thiamine disulfide dimyristate.

## BEST MODE FOR CARRYING OUT THE INVENTION

No particular limitation is imposed on the salt of the compound (1) according to the present invention so far as it is a pharmaceutically acceptable salt. Examples thereof include acid-addition salts, particularly, salts with inorganic acids such as hydrochloric acid, nitric acid and sulfuric acid, and salts with organic acids such as acetic acid and succinic acid. The compound (1) according to the present invention may be present in the form of solvates such as hydrates and form complexes with other components.

The compound (1) according to the present invention can be prepared by allowing thiamine disulfide to react with myristic acid or a reactive derivative thereof in accordance with, for example, the following reaction scheme:

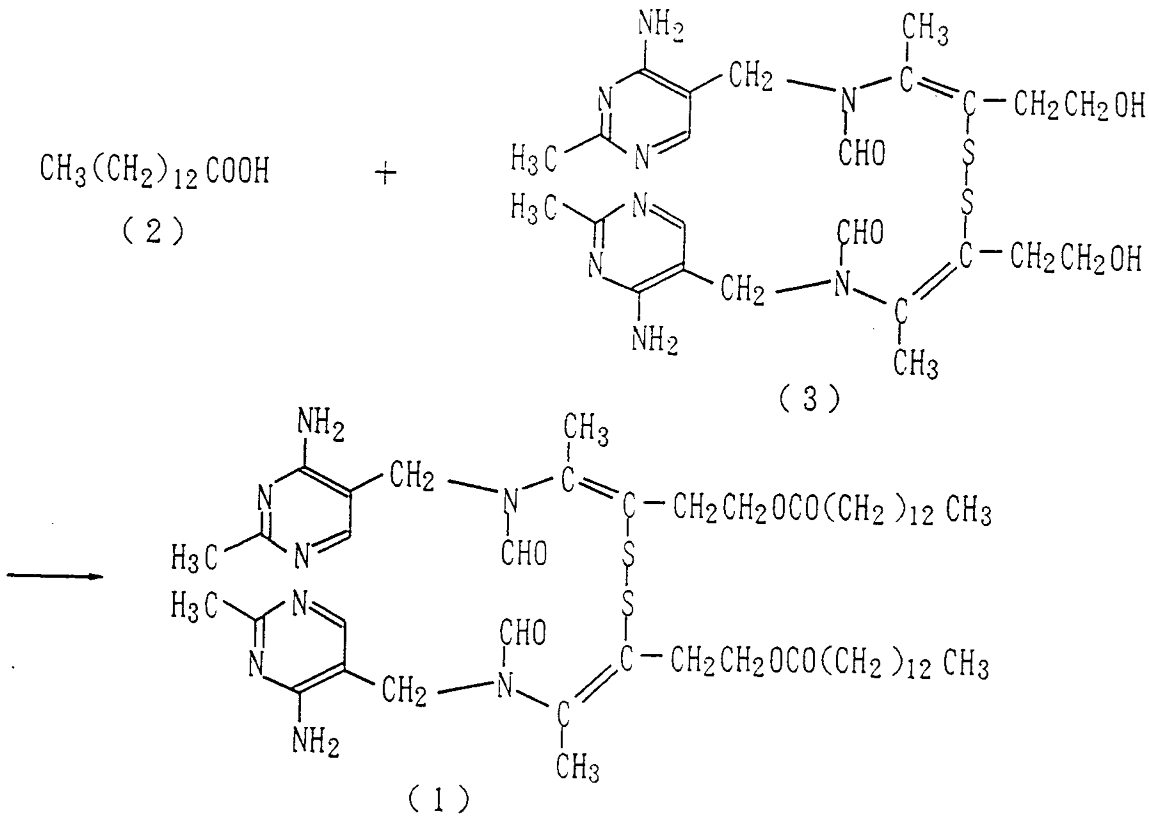

(2)

(3)

(1)

Examples of the reactive derivative of myristic acid, which is used in the reaction, include acid halides such as acid chloride and acid bromide, and acid anhydride. When myristic acid is allowed to directly react, it is preferable to use a condensing agent such as dicyclohexylcarbodiimide.

The reaction is preferably conducted either in a basic solvent such as pyridine or in the presence of a base such as pyridine or N,N-dimethylaniline in a solvent such as chloroform or benzene. The reaction temperature may be generally room temperature, and the reaction time may be generally about 1 to 10 hours.

The compound (1) according to the present invention or the salt thereof has excellent anti-HIV activity against cells persistently infected with HIV and is useful as a therapeutic agent for AIDS.

The anti-HIV agent, and prophylactic and therapeutic agent for AIDS according to the present invention are obtained by suitably adding pharmaceutically acceptable carriers such as an excipient, binder, lubricant, disintegrator, coating, emulsifier, suspending agent, solvent, stabilizer, absorbefacient and ointment base to the compound (1) or the salt thereof as needed, or forming a liposome thereof, thereby formulating a preparation in a form for oral administration, injection, intrarectal administration or the like in accordance with a method known *per se* in the art.

The preparation for the oral administration may preferably be in the form of a granule, tablet, sugar-coated tablet,

capsule, soft capsule, pill, solution, emulsion, suspension or the like; the preparation for the injection administration may preferably be in the form for intravenous injection, intramuscular injection, subcutaneous injection, drip injection or the like; and the preparation for the intrarectal administration may preferably be in the form of a suppository, capsule or the like.

The dose of such a preparation varies according to administration route, the age and condition of a patient to be administered, and the like. However, it is preferable that the dose be generally 1 to 1,000 mg per day for an adult in terms of the compound (1) or the salt thereof. This amount of the preparation is administered once or in portions.

The anti-HIV agent, and prophylactic and therapeutic agent for AIDS according to the present invention may be used in combination with other anti-HIV agents typified by azidothymidine and didanosine. These other anti-HIV agents may be incorporated into the agents according to the present invention.

## EXAMPLES

The present invention will hereinafter be described in more detail by the following examples. However, the present invention is not limited to these examples.

Example 1:

Thiamine disulfide (4.06 g) was dissolved in dry pyridine (40 ml). Myristoyl chloride (3.91 g) was added to the resultant solution, and the mixture was stirred at room temperature for 3.5 hours.

After completion of the reaction, the reaction mixture was dispersed in ice water, and white crystals formed were collected by filtration. The thus-obtained crystals were dissolved in chloroform and purified by column chromatography on silica gel. Elution was conducted with a mixture of chloroform and methanol (chloroform:methanol = 9:1) to obtain 3.34 g (yield: 40.7%) of thiamine disulfide dimyristate as a pale yellow oil.

IR (neat) $\nu$ $cm^{-1}$: 3344, 3200, 2932, 2860, 1898, 1740, 1668, 1596, 1558, 1518.
NMR: Illustrated in Fig. 1.

Test Example 1: (Anti-HIV activity)

A test agent was added at predetermined concentrations to cells (2 x $10^5$ cells/ml, 10 ml) persistently infected with HIV in a logarithmic growth phase to culture the cells for 96 hours. The number of viable cells and the number of killed cells were counted every 24 hours by a trypan blue dye exclusion test to determine the cytotoxic effect of the test agent on the cells. A viral infectious titer ($TCID_{50}$/ml) in each of the culture solutions incubated for 96 hours was determined by 72-hour culture using, as an index, the giant cell formation of MT-4 cells. In a control group, the cells were cultured in a medium free of any agent. An anti-HIV activity (inhibition %) was determined in accordance with the following equation:

$$\text{Anti-HIV activity (inhibition \%)} = \frac{TC}{TC} \times 100$$

As a result, as shown in Table 1, the compound (1) according to the present invention exhibited marked anti-HIV activity against the cells, or CEM/LAV-1, which persistently produced HIV, at a concentration of from 50 to 125 μm, and its inhibitory effect was almost 100%.

| Invention compound (μM) | $TCID_{50}$/ml | Inhibition rate (%) |
|---|---|---|
| 0 (Control) | 4 x $10^5$ | - |
| 50 | 8 x $10^2$ | 99.5 |
| 125 | 8 x $10^2$ | 99.5 |

## INDUSTRIAL APPLICABILITY

The compound (1) according to the present invention or the salt thereof has an excellent anti-HIV effect on cells persistently infected with HIV and is useful as a prophylactic and therapeutic agent for AIDS.

## Claims

1. Thiamine disulfide dimyristate represented by the following formula (1):

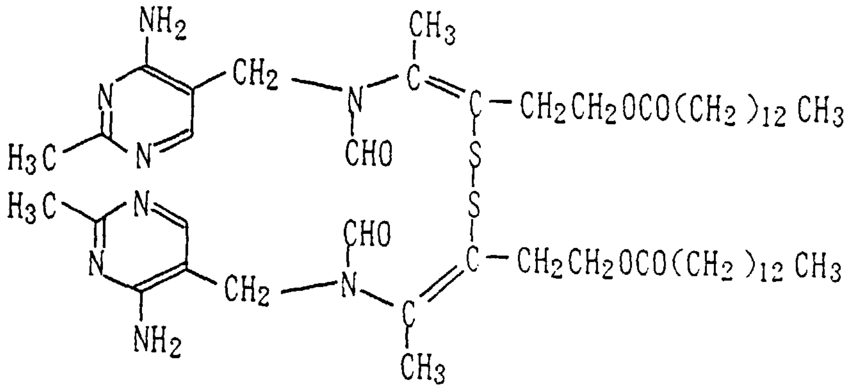

(1)

or a salt thereof.

2. An anti-HIV agent comprising thiamine disulfide dimyristate (1) or the salt thereof according to Claim 1 as an active ingredient.

3. A prophylactic and therapeutic agent for acquired immune deficiency syndrome, comprising thiamine disulfide dimyristate (1) or the salt thereof according to Claim 1 as an active ingredient.

4. An anti-HIV agent composition comprising thiamine disulfide dimyristate (1) or the salt thereof according to Claim 1 and a pharmaceutically acceptable carrier.

5. A prophylactic and therapeutic agent composition for acquired immune deficiency syndrome, comprising thiamine disulfide dimyristate (1) or the salt thereof according to Claim 1 and a pharmaceutically acceptable carrier.

6. Use of thiamine disulfide dimyristate (1) or the salt thereof according to Claim 1 for the preparation of an anti-HIV agent.

7. Use of thiamine disulfide dimyristate (1) or the salt thereof according to Claim 1 for the preparation of a prophylactic and therapeutic agent for acquired immune deficiency syndrome.

8. A method for the treatment of a disease caused by infection with HIV, which comprises administering an effective amount of thiamine disulfide dimyristate (1) or the salt thereof according to Claim 1 to a patient to be treated.

9. A method for the treatment of acquired immune deficiency syndrome, which comprises administering an effective amount of thiamine disulfide dimyristate (1) or the salt thereof according to Claim 1 to a patient to be treated.

Fig. 1

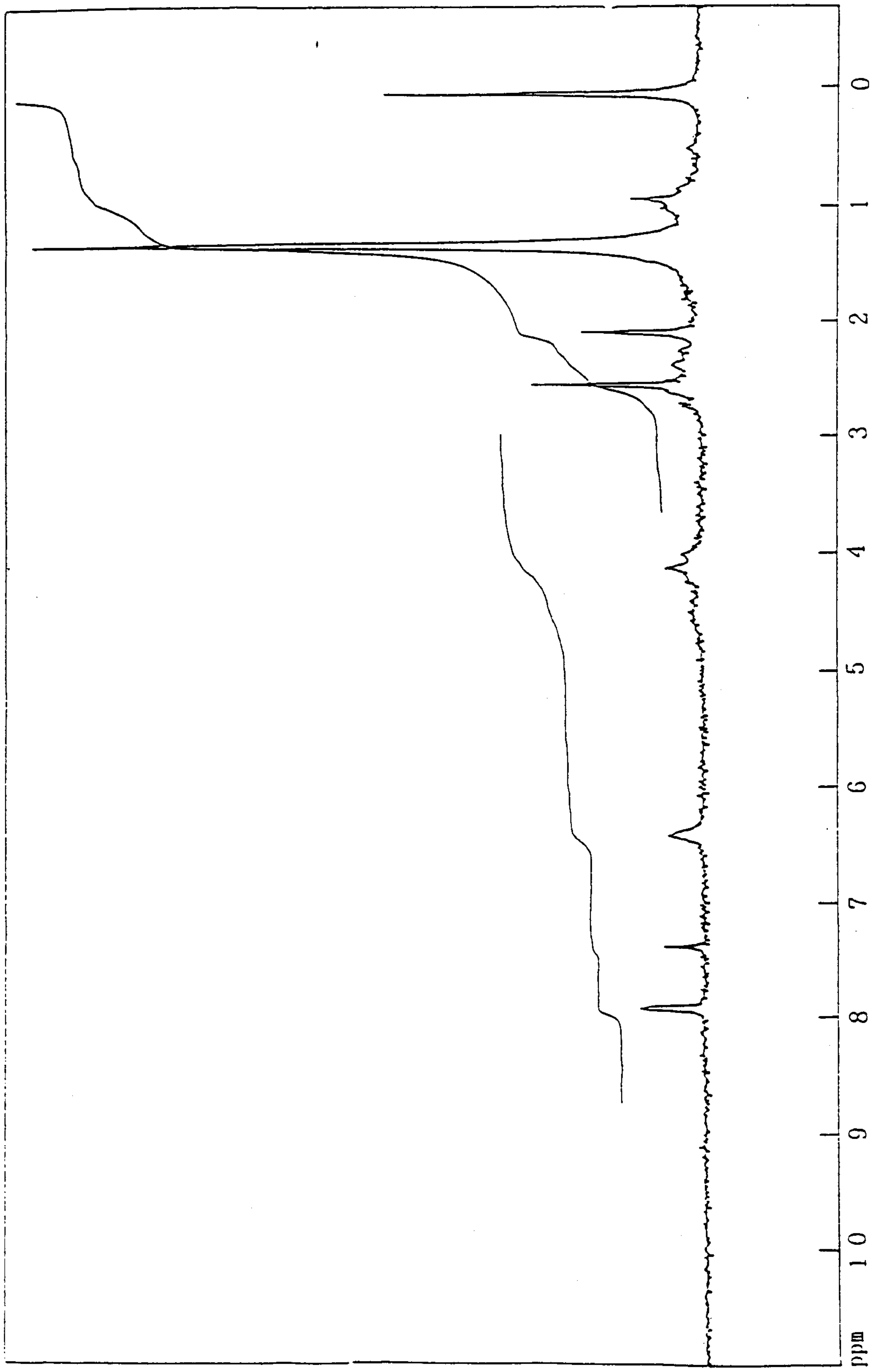

**INTERNATIONAL SEARCH REPORT**

International application No. PCT/JP96/03341

**A. CLASSIFICATION OF SUBJECT MATTER**

Int. $Cl^6$ A61K31/505, C07D239/42

According to International Patent Classification (IPC) or to both national classification and IPC

**B. FIELDS SEARCHED**

Minimum documentation searched (classification system followed by classification symbols)

Int. $Cl^6$ A61K31/505, C07D239/42

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| X | WO, 9520388, A (Nissui Pharmaceutical Co., Ltd.), August 3, 1995 (03. 08. 95), Claim & JP, 7-519779, A | 1 - 7 |

☐ Further documents are listed in the continuation of Box C. ☐ See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| February 3, 1997 (03. 02. 97) | February 12, 1997 (12. 02. 97) |

| Name and mailing address of the ISA/ | Authorized officer |
|---|---|
| Japanese Patent Office | |
| Facsimile No. | Telephone No. |

Form PCT/ISA/210 (second sheet) (July 1992)

## INTERNATIONAL SEARCH REPORT

International application No.
PCT/JP96/03341

**Box I Observations where certain claims were found unsearchable (Continuation of item 1 of first sheet)**

This international search report has not been established in respect of certain claims under Article 17(2)(a) for the following reasons:

1. [X] Claims Nos.: 8, 9
because they relate to subject matter not required to be searched by this Authority, namely:
Claims 8 and 9 pertain to methods for treatment of the human or animal body by therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest.
[ ] No protest accompanied the payment of additional search fees.

Form PCT/ISA/210 (continuation of first sheet (1)) (July 1992)